(19) Europäisches Patentamt
European Patent Office
Office européen des brevets

(11) Publication number: **0 367 145**
**A2**

(12) # EUROPEAN PATENT APPLICATION

(21) Application number: 89120010.7

(22) Date of filing: 27.10.89

(51) Int. Cl.5 C12P 9/00 , C07F 9/02 , //A01N57/20

(30) Priority: 27.10.88 JP 269541/88

(43) Date of publication of application: 09.05.90 Bulletin 90/19

(84) Designated Contracting States: DE FR GB IT

(71) Applicant: MEIJI SEIKA KAISHA LTD.
4-16 Kyobashi 2-chome
Chuo-ku Tokyo(JP)

(72) Inventor: Imai, Satoshi Pharmaceutical Research-Center
Meiji Seika Kaisha Ltd. 760 Morooka-cho
Kohoku-ku
Yokohama-shi Kanagawa-ken(JP)
Inventor: Takahashi, Makoto Pharmaceutical Research-Center
Meiji Seika Kaisha Ltd. 760 Morooka-cho
Kohoku-ku
Yokohama-shi Kanagawa-ken(JP)
Inventor: Fukatsu, Shunzo Pharmaceutical Research-Center
Meiji Seika Kaisha Ltd. 760 Morooka-cho
Kohoku-ku
Yokohama-shi Kanagawa-ken(JP)
Inventor: Ogawa, Yasuaki c/o Meiji Seika Ltd.
4-16, Kyobashi 2-Chome
Chuo-ku Tokyo(JP)

(74) Representative: Sajda, Wolf E., Dipl.-Phys. et al
MEISSNER, BOLTE & PARTNER
Widenmayerstrasse 48 Postfach 86 06 24
D-8000 München 86(DE)

(54) **New process for the production of L-2-amino-4(hydroxymethyl-phospinyl)-butyric acid.**

(57) A known herbicidal compound, L-2-amino-4-(hydroxymethylphosphinyl)-butyric acid can be produced by a new process comprising transaminating 4-(hydroxymethylphosphinyl)-2-oxo-butyric acid by reacting it with fumaric acid and ammonium ion in the presence of an aspartase and a transaminase which are produced by a microorganism capable of producing both said aspartase and transaminase, or alternatively in the presence of cells of said microorganism or a purified or partially purified enzyme preparation as prepared from said cells and containing the aspartase and transaminase.

EP 0 367 145 A2

# New process for the production of L-2-amino-4-(hydroxymethyl-phosphinyl)-butyric acid

## Summary of the Invention

This invention relates to a new process for the production of L-2-amino-4-(hydroxymethylphosphinyl)-butyric acid which exhibits herbicidal activities and is known to be useful as a herbicide (see Japanese patent publication No. 56210/86 specification or U.S. patent No. 4,265,654 specification).

## Background of the Invention

L-2-Amino-4-(hydroxymethylphosphinyl)-butyric acid having the formula

$$H_3C-\underset{OH}{\underset{|}{\overset{O}{\overset{\|}{P}}}}-CH_2-CH_2-\underset{NH_2}{\underset{|}{CH}}-COOH \qquad (I)$$

is a known compound called as phosphinothricin and shows a herbicidal activity and is useful as a herbicidal agent. The known methods for the production of L-2-amino-4-(hydroxymethylphosphinyl)-butyric acid (hereinafter abbreviated as "L-AMPB") include such a method wherein an antibiotic substance SF-1293 namely, L-2-amino-4-(hydroxymethylphosphinyl)-butyryl-L-alanyl-L-alanine (see U.S. patent No. 4,309,208) is subjected to hydrolysis (see Japanese Patent Application first publication "Kokai" No. 85538/73), and such a method wherein the SF-1293 substance is decomposed with a microbial enzyme (see Japanese Patent Application first publication "Kokai" No. 31890/74). Besides, there is known method wherein AMPB in the form of a racemic mixture is produced by a chemical synthetic process (see Japanese Patent Application first publication "Kokai" Nos. 91019/73 and 84529/79) and then the racemic AMPB product is subjected to optical resolution with aid of a microbial enzyme to yield L-AMPB. In addition, there is known a method wherein an L-AMPB-producing strain of the genus Streptomyces such as Streptomyces hygroscopicus is cultivated under aerobic conditions, followed by recovering L-AMPB directly from the resulting culture broth (see Japanese Patent publication No. 8760/88). Furthermore, there is known a further method for producing L-AMPB in large quantities wherein 4-(hydroxymethylphosphinyl)-2-oxo-butyric acid as chemically synthesized is used as a substrate and is transaminated with an amino acid acting as an amino-donor in the presence of a transaminase as produced by a microorganism, so that the substrate compound is converted into L-AMPB through the enzymatic reaction (see European patent application publication No. 0249188A2 or its corresponding U.S. patent application SN.07/058,872. 4-(hydroxymethylphosphinyl)-2-oxo-butyric acid (hereinafter abbreviated as "OMPB") used as the substrate in the above-mentioned method is a known compound which is produced by a process described in Japanese patent application first publication "Kokai" No. 92897/81 or U.S. patent No. 4,399,287 specification. On the other hand, it is known that 3,4-dihydroxyphenyl-L-alanine can be produced by transamination of its corresponding keto acid, namely 3,4-dihydroxyphenyl-pyruvic acid with an amino-donor (namely, amino-radical-donor) selected from several amino acids, in the presence of a transaminase which is produced by some kinds of bacteria, molds and yeasts and which is capable of effecting the transamination between 3,4-dihydroxyphenyl-pyruvic acid and the amino acid acting as the amino-donor (see U.S. patent No. 3,767,528) and that L-4-phenyl-2-aminobutanoic acid can be produced by transamination of 4-phenyl-2-oxobutanoic acid with L-aspartic acid as the amino-donor in the presence of a bacterial transaminase enzyme (see U.S. patent No. 4,525,454).

When a herbicidal compound is to be provided commercially, it is at first necessary that a method which can produce said herbicidal compound at a large scale is available and also it is essentially necessary that said herbicidal compound can be produced at low cost. With the above-mentioned method for the production of L-AMPB wherein the chemically synthesized OMPB is transaminated in the presence of a microbial transaminase enzyme with using one or more amino acids as the amino-donor, however, it is obvious that the cost of the amino acids such as glutamic acid and aspartic acid which must be used as the amino-donor can greatly affect and increase the cost for the production of L-AMPB to be produced through said method. Accordingly, a demand is existing that the method for producing L-AMPB where OMPB is

transaminated with amino-donor in the presence of a transaminase should be so modified or improved that the said method is made workable more economically with reduced costs.

In order to provide a more economical method for producing L-AMPB, we, the present inventors, have made researches in an attempt to discover such amino-donor compounds which are much more cheaper than the amino acids but are utilizable as a substitute for said amino acid in the method of producing L-AMPB where the enzymatic transamination of OMPB with an amino-donor is involved. Previously, we discovered that L-AMPB can be produced by reacting OMPB with NADH and ammonium chloride in the presence of a glutamic acid-dehydrogenase (GLDH) to effect the transamination between OMPB and the combination of NADH and ammonium chloride (see Example 1 of the aforesaid European patent application publication No. 0249188A2 and its corresponding, pending U.S. patent application SN.07/058,872). However, it is actual that when OMPB is reacted with NADH and ammonium chloride in the presence of GLDH, the rate of conversion of OMPB into L-AMPB is disadvantageously very much lower than when OMPB is transaminated with an amino acid as the amino-donor in the presence of the microbial transaminase enzyme. In our further researches, we have tested various kinds of microorganisms as well as many organic compounds and inorganic compounds which will possibly be utilizable in the method of producing L-AMPB comprising the enzymatic transamination of OMPB. As a result, we have now surprisingly found that when OMPB is reacted with inexpensive fumaric acid and ammonium ion in the presence of such a microorganism which is capable of producing an aspartase and also capable of producing a transaminase, OMPB can be converted into L-AMPB at an increased rate, and we have discovered that fumaric acid and ammonium ion in combination are then able to promote the enzymatic transamination of OMPB into L-AMPB in the presence of both an aspartase and a transaminase which are produced by said microorganism. In this process, it is not needed that the aspartase and transaminase as produced by said microorganism are isolated into a partially purified or purified enzyme preparation, but the microbial aspartase and transaminase may be effective for the intended purpose even when they are remaining in a crude form, for example, in the form of a culture broth of said microorganism, separated intact cells of said microorganism, disrupted cells of said microorganism, and a crude enzyme solution containing the enzymes as extracted from the disrupted cells and other partially purified enzyme preparations. Of course, the aspartase and transaminase may be used in the form of their purified enzyme preparations.

While, we are aware of that there has been reported a method for producing L-phenylalanine, a naturally-occuring amino acid known as a constituent amino acid of proteins, wherein L-phenylalanine may be produced either by cultivating in a culture medium in the presence of phenylpyruvic acid as well as fumaric acid and ammonium ion or urea such a microorganism of the genus Arthrobacter, Bacillus, Brevibacterium, Corynebacterium, Flavobacterium, Klebsiella, Kluyvera, or Micrococcus which is capable of converting phenylpyruvic acid into L-phenylalanine in the presence of fumaric acid and ammonium ion or urea, or by reacting cultured cells of said microorganism or disrupted cells thereof or a lyophillized product of said disrupted cells, or a proteinous fraction of said cells or the like with an aqueous liquid containing phenylpyruvic acid as well as fumaric acid and ammonium ion or urea (Japanese patent application first publication "Kokai" No. 141893/86), and it is described there that Arthrobacter globiformis ATCC 8010, Bacillus sphaericus ATCC 10208, Brevibacterium lactofermentum ATCC 13655, Corynebacterium hydrocarboclastum ATCC 15108, Flavobacterium suaneolens ATCC 958, Klebsiella oxytoca ATCC 8724, Kluyvera cryorescens ATCC 14237 and Micrococcus luteus ATCC 10240 are illustrated as available examples of said microorganism having the capacity required in this prior art method just mentioned in the above, but there is not given any description that the microorganism to be used in this prior art method must have an activity of aspartase.

Nonetheless, as long as we know, it is unexpectable that fumaric acid and ammonium ion in combination are able to promote the transamination of OMPB having its phosphorus-containing unique structure where the linkage C-P-C exists so that OMPB is converted into L-AMPB which is only one natural amino acid where the unique linkage C-P-C exists,and in this aspect L-AMPB is evidently distinguishable from the ordinary constituent amino acids of proteins. On the basis of our findings as above, we have now accomplished the present invention to provide an economical and efficient process of producing L-AMPB wherein OMPB is used as the substrate and reacted with fumaric acid and ammonium ion in the presence of a microorganism which is capable of producing both an aspartase and a transaminase or in the presence of a purified or partially purified enzyme preparation containing the aspartase and transaminase as extracted from the cells of said microorganism.

## Detailed description of the invention

In a first aspect of this invention, therefore, there is provided a process for the production of L-2-amino-4-(hydroxymethylphosphinyl)-butyric acid of the formula (I):

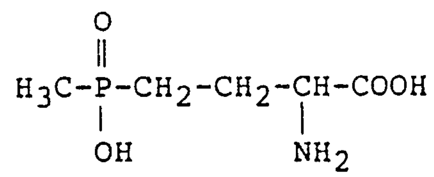

(I)

which comprises transaminating 4-(hydroxymethylphosphinyl)-2-oxo-butyric acid of the formula (II):

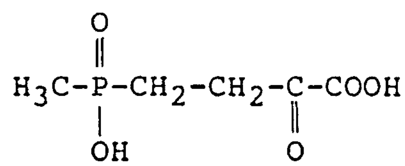

(II)

by reacting with fumaric acid and ammonium ion either in the presence of a culture broth of a microorganism which is capable of producing an aspartase and also capable of producing a transaminase that can effect transamination between 4-(hydroxymethylphosphinyl)-2-oxo-butyric acid and an amino-donor to yield said L-2-amino-4-(hydroxymethylphosphinyl)-butyric acid, or in the presence of separated intact cells of said microorganism, or in the presence of a purified or partially purified enzyme preparation containing said aspartase and transaminase as extracted from the cells of said microorganism.

The microorganism usable according to this invention may be any of such microorganisms which are capable of producing an aspartase and also capable of producing the transaminase and which are able to produce L-AMPB when OMPB is reacted with fumaric acid and ammonium ion in the presence of that microorganism. Aspartase (L-aspartate ammonia-lyase) [EC4.3.1.1] is an enzyme which catalizes the reversible conversion of L-aspartate to fumarate and ammonium ion in aqueous solution. In the process of this invention, it is possible to use an aspartase-producing and transaminase-producing microorganism which is taken from the group of the genera Escherichia, Pseudomonas, Klebsiella, Brevibacterium, Serratia, Proteus and Bacillus. Such aspartase-producing and transaminase-producing microorganisms are readily available and distributable from the public depositories such as ATCC (namely, "American Type Culture Collection" located in Maryland, U.S.A.), NCTC (namely, "National Collection of Type Culture" located in London, U.K.), IAM (namely, "Institute of Applied Microbiology, University of Tokyo" located in Tokyo, Japan), NIHJ (namely, "National Institute of Health, Ministry of Health and Welfare" located in at Shinagawa-ku,Tokyo, Japan) and IFO (namely "Institute for Fermentation, Osaka" located in Osaka, Japan), etc.

Suitable examples of the microorganisms for use in the process of this invention include Escherichia coli NCTC 8196 and Pseudomonas aeruginosa NCTC 10490 which have been deposited in the depository "NCTC" and are distributable to the public therefrom ; Escherichia coli NIHJ JC-2 (IFO 12734) which has been deposited in the depository "NIHJ" or "IFO" and is distributable to the public therefrom ; Serratia marcescens IAM 1223 which has been deposited in the depository "IAM" and is distributable to the public therefrom ; and Klebsiella pneumoniae ATCC 10031, Proteus vulgaris ATCC 13315 and Bacillus subtilis ATCC 6633 which have been deposited in the depository "ATCC" and are distributable to the public therefrom. Or alternatively, any persons skilled in the art will be able to readily isolate newly and obtain such aspartase-producing and transaminase-producing microorganisms from the naturally-occurring bacterial strains by investigating their activity of aspartase and their ability of producing L-AMPB from the substrate OMPB in the presence of fumaric acid and ammonium ion. The presence of aspartase activity in a bacterial strain can conveniently be confirmable by a method comprising reacting said bacterial strain with L-aspartate and measuring the change in absorbance at 240 mμ for showing the formation of fumarate in the reaction solution (see "Method in Enzymology" 13, 354 (1969)).

Any aspartase-producing and transaminase-producing microorganism, even if it is such one newly isolated from the nature, can be used in this invention, provided that it is capable of producing L-AMPB from OMPB as substrate in the presence of fumaric acid and ammonium ion. The properties of the microorganisms usable in this invention are easy to change, as is often seen with other bacterial strains. For

example, they can be artificially mutated by means of ultraviolet rays, X-rays, chemical agents, etc. Any of such mutants may be used in the invention if it is capable of producing L-AMPB from the substrate OMPB in the presence of fumaric acid and ammonium ion. Genes of enzymes, such as aminotransferases (namely, transaminase) and aspartases, have been cloned according to the known genetic engineering techniques and hence can be introduced into microorganisms for their transformation. Any microorganism which has been transformed with such genes may also be used in the invention if it is capable of producing L-AMPB from the substrate OMPB in the presence of fumaric acid and ammonium ion.

The microorganisms usable in the process of this invention can be cultured in a culture medium containing nutrients which ordinary microorganisms can assimilate. Any nutrients ordinarily employed for the cultivation of microorganisms can be used for this purpose. Examples of assimilable carbon sources include glucose, fructose, sucrose, starch, glycerol, fumaric acid, citric acid, malic acid, starch syrup, molasses, and the like. These can be used individually, or two or more of them can be used in combination. Examples of assimilable nitrogen sources include peptone, yeast extract, corn steep liquor, meat extract, soy bean powders, wheat germ, ammonium sulfate, ammonium chloride, sodium nitrate and mixtures of these. If required, the culture medium can be incorporated with inorganic salts, such as calcium carbonate, magnesium sulfate, sodium chloride, potassium chloride, phosphates, etc., as well as other organic and inorganic substances which could promote the formation of L-AMPB from the substrate OMPB. It can be particularly effective to cultivate the microorganism to be used in the present invention in such a culture medium which has been incorporated with aspartic acid and/or fumaric acid that are known to function as an inducer for aspartase activities in the microorganism. The microorganisms to be used in the process of the invention can be cultured in accordance with known methods conventionally used in the cultivation of ordinary microorganisms. Culture medium, cultivation temperature, etc. suited for their cultivation are described in the catalogues issued by ATCC and other depositories. Cultivation time can readily and suitably be decided according to the growth of microorganisms used, their aspartase activities, and their ability for producing L-AMPB from the substrate OMPB. In the process of this invention, the transamination reaction of OMPB can not only be effected in the presence of a culture broth of the available microorganism, but also can be effected in an aqueous suspension containing intact cells of the microorganism as separated from said culture broth. Further, this reaction can also be effected in the presence of such a purified or a partially purified enzyme preparation containing the necessary aspartase and transaminase that is extracted from the cells of said microorganism and may be prepared by disrupting the cells by sonication or treating the cells with lysozyme. Furthermore, it is known that the stability and operability of the microorganism and the enzymes can be improved by immobilizing them with organic solvents, cross-linking agent, carrier or the like. The microorganism and the enzymes usable in the process of this invention which have been immobilized by known immobilization methods can be used effectively in the process of this invention.

When the process of this invention is carried out, it is preferred that fumaric acid is present in the reaction mixture in an amount in a range of from an equimolar proportion to 4 molar proportion per 1 molar proportion of the substrate OMPB, and that ammonium ion is provided usually in the form of aqueous ammonia in an amount required to neutralize fumaric acid present in the reaction mixture. Thus, ammonium ion may preferably be present in the reaction mixture at least in an proportion equimolar to the molar proportion of fumaric acid present. It is also possible that ammonium ion is provided by adding an aqueous solution of urea which usually decomposes into ammonia. In general, the reaction according to the process of this invention may suitably be carried out at a temperature of 30 °C to 70 °C and under alkaline conditions in a range of pH 8 to pH 10 for a time of 15 hours to 48 hours until a substantial amount of L-AMPB is produced and accumulated in the reaction solution. Optimal reaction temperature and optimal pH conditions vary according to the nature of the microorganism if this is employed but they can readily be decided by the skilled in the art so as to ensure that the yield of the desired product, L-AMPB can reach a maximum.

As a result of our further study, we have now found that the yield of L-AMPB can further be improved considerably when the transamination reaction of OMPB in the presence of fumaric acid and ammonium ion and in the presence of the microorganism or in the presence of the enzymes as extracted from said microorganism is carried out in the additional presence of glutamic acid added, especially L-glutamic acid or its sodium salt which is known to be a best substrate for the transaminase. Suitable amount of glutamic acid added can vary according to the nature of the microorganism as employed and the reaction conditions for carrying out the process, including the conditions for incubation of the microorganism as employed. In general, however, it is suitable that glutamic acid added is present in the reaction mixture at most in a proportion equimolar to the molar proportion of the substrate OMPB present. From the view-point of economic, it is preferred that glutamic acid is added in a molar proportion of 10% to 50% based on one

molar proportion of OMPB.

According to a particular embodiment of this invention, there is provided a process for producing L-AMPB which comprises mixing OMPB and water with fumaric acid and ammonium ion and also with intact cells of the aspartase-producing and transaminase-producing microorganism or a purified or partially purified enzyme preparation containing the aspartase and transaminase of said microorganism, optionally together with L-glutamic acid or sodium L-glutamate, and then incubating the resulting mixture at a temperature of 30°C to 70°C at pH8 to pH10 until OMPB is transaminated to produce and accumulate L-AMPB in the incubated mixture,and then recovering L-AMPB from said incubated mixture. In this embodiment of this invention, the proportions of OMPB, fumaric acid, ammonium ion and the glutamic acid compound used are same as those described hereinbefore for the process of the first aspect of this invention. The conditions for the incubation of said resulting mixture are also same as those conditions for the transamination of OMPB described hereinbefore.

Still,when the transamination reaction of OMPB is effected in the presence of a culture broth of the aspartase-producing and transaminase-producing microorganism or in the presence of the separated intact cells of said microoraganism according to the process of this invention, it can be assumed that the transamination reaction of OMPB to yield L-AMPB proceeds under the enzymatic actions of the aspartase and transaminase in combination which are produced by said aspartase-producing and transaminase-producing microorganism employed. In another aspect of this invention when expressed more concisely, there is provided a process for the production of L-AMPB, which comprises transaminating OMPB by reacting with fumaric acid and ammonium ion together with or without L-glutamic acid or its sodium salt in the presence of an aspartase and a transaminase which are produced by an aspartase-producing and transaminase-producing microorganism selected from the groups of bacterial strains of the genera Escherichia, Pseudomonas, Klebsiella, Brevibacterium, Serratia, Proteus and Bacillus, thereby to produce L-2-amino-4-(hydroxymethylphosphinyl)-butyric acid.

Now, the recovery of the L-AMPB product from the reaction solution which contains L-AMPB as produced according to the process of this invention is now described. Recovery and purification of L-AMPB from the reaction solution obtained may be conducted in the same manner as in the known methods of recovering and purifying L-AMPB from the culture broth of the L-AMPB-producing microorganism which is obtained according to the known fermentative method of producing L-AMPB. Detailed procedures for recovery and purification of L-AMPB are disclosed in the aforesaid Japanese patent publication No. 8760/88. For instance, the recovery and purification of L-AMPB from the reaction solution obtained from the process of this invention may be achieved by passing the L-AMPB-containing reaction solution through a column of a cation-exchange resin such as Dowex 50 W (product of Rohm & Haas Co., Ltd., U.S.A.) to make L-AMPB adsorbed by this resin, and then eluting the resin column with water or diluted aqueous ammonia solution to give such fractions of the eluate containing L-AMPB. Otherwise, L-AMPB may be adsorbed by an anion-exchange resin such as Dowex 1 x 2, followed by eluting with aqueous acetic acid. The eluate fractions containing L-AMPB may then be collected together and concentrated under reduced pressure to afford a dry powder of L-AMPB. The L-AMPB as produced according to the process of this invention shows the same physical and chemical properties as those of an authentic sample of L-AMPB which has been afforded by the known fermentative method disclosed in the Japanese patent application publication No. 8760/88, and also it is observed that the L-AMPB produced according to this invention shows the same herbicidal activities as those of the L-AMPB produced by the known fermentative method when tested by some herbicidal methods.

This invention is now illustrated with reference to the following Examples to which this invention is not limited.

## Example 1

A culture medium comprising 1.2% sodium L-aspartate, 0.5% peptone, 0.3% yeast extract, 0.2% dipotassium phosphate and 0.05% magnesium sulfate (pH 7.2) was placed in 30 mℓ-aliquots in conical flasks. One loopful quantity of one of the microorganisms indicated in Table 1 below was inoculated into the culture medium in each flask, followed by shake-cultivating the inoculated bacterial strain at 37°C at a rotation speed of 220 rpm. overnight. The resulting culture broth was centrifuged to collect the intact cells of the cultured microorganism. The intact cells thus separated were suspended in 100 mM Tris-hydrochloride buffered solution (pH 8.5) to prepare a cell suspension.

To this cell suspension OMPB, an aqueous solution of fumaric acid as neutralized with aqueous ammonia, and sodium L-glutamate were added so as to prepare 6 mℓ of a reaction mixture where the final

concentrations of OMPB, ammonium fumarate and sodium L-glutamate were adjusted to 100 mM, 200 mM and 20mM, respectively. The resulting reaction mixture was poured in portions into L-shaped glass tubes, followed by incubating the reaction mixture at 37° C for 24 hours at a rotation speed of 100 rpm. to effect the transamination of OMPB. After the completed reaction, the resultant reaction solution was adjusted to pH 3 with addition of hydrochloric acid and then centrifuged to afford a supernatant solution containing L-AMPB as produced. The reaction product L-AMPB present in the supernatant solution was quantatively analyzed using an amino acid analyzer.

On the other hand, a further 3 mℓ-aliquot of the cell suspension as prepared in the same way as above was taken and the cells were disrupted by sonication in Kubota Insonator, model-200 M, at 1.5 Ampers, for 3 minutes to prepare a liquid mixture comprising the disrupted cells. This mixture was then centrifuged to afford an enzyme extract solution containing the enzymes as extracted from the microbial cells. This enzyme extract solution was assayed for its activity of aspartase in accordance with a method described in the "Method in Enzymology" 13, 354 (1969). The test results obtained are tabulated in Table 1 below. Comparison of the test results reveal that in general, a fair co-relationship exists between the degree of the aspartase activity of the enzyme extract solution (and hence the aspartase activity of the microbial strain employed) and the amount of L-AMPB produced.

Table 1

| Microorganisms Tested | Aspartase Activity (units/mℓ) | Amount of L-AMPB produced (mM) |
|---|---|---|
| *Escherichia coli* NCTC 8190 | 12.0 | 80.0 |
| *Escherichia coli* NIHJ JC-2 (IFO 12734) | 12.5 | 71.8 |
| *Pseudomonus aeruginosa* NCTC 10490 | 2.2 | 81.7 |
| *Klebsiella pneumoniae* ATCC 10031 | 1.2 | 13.9 |
| *Seratia marcescens* IAM 1223 | 0.1 | 7.6 |
| *Proteus vulgaris* ATCC 13315 | 2.2 | 11.2 |
| *Bacillus subtilis* ATCC 6633 | 7.7 | 43.2 |

## Example 2

(1) A culture medium comprising 1.0% Bactotrypsin (a product of Difco Co.), 0.5% yeast extract (a product of Difco Co.), 0.5% sodium chloride and 0.1% glucose (namely, L-broth medium at pH 7.0) was placed in 40 mℓ-aliquots in conical flasks of 250 mℓ-capacity. One loopful quantity of *Escherichia coli* NCTC 8196 was inoculated into the culture medium in each flask, followed by shake-cultivating the inoculated bacterial strain at 37° C at a rotation speed of 220 rpm. overnight to give a seed culture. This seed culture was inoculated into 2ℓ of a culture medium comprising about 3% ammonium fumarate, 2% corn steep liquor, 0.2% dipotassium phosphate, 0.05% magnesium sulfate and 0.05% calcium carbonate (pH 7.0) which had been charged in a jar-fermentor of 3ℓ -capacity. The cultivation of the bacterial strain inoculated was then conducted for 16 hours under shaking and aeration. The resulting culture broth (1.86ℓ ) was then centrifuged and the intact cells collected were washed with 0.1M-Tris-hydrochloride buffered solution (pH 8.5) to obtain 17.08 g of wet intact cells of *E. coli* NCTC 8196.

An amount (0.467 g) of the cells was taken and suspended in 100 mM Tris-hydrochloride buffered solution (pH 8.5) to prepare the cell suspension. To this cell suspension OMPB, aqueous neutralammonium fumarate, and sodium L-glutamate were added so as to prepare 10 mℓ of a reaction mixture wherein the final concentrations of OMPB, ammonium fumarate and sodium L-glutamate were adjusted to the respective concentrations as indicated in Table 2 below. In some tests, however, sodium L-glutamate or fumarate was not added. The resulting reaction mixture was placed in a conical flask of 100 mℓ-capacity and then incubated at 37° C, 45° C or 60° C for 22 hours under varying pH conditions and under agitation at 200 rpm. to effect the transamination of OMPB. After the completed reaction, the resulting reaction solution was analysed to determine its content of L-AMPB produced therein. The test results obtained are tabulated in Table 2 below. By making comparisons between the test results of Table 2, it is shown that L-AMPB is produced from OMPB by the transamination of OMPB which took place in the presence of fumaric acid and

ammonium ion and in the presence of the cells of the bacterial strain as employed, and that the yield of L-AMPB produced can be increased by addition of L-glutamic acid (in the form of sodium L-glutamate) in the above transamination process of OMPB.

Table 2

| Concentrations (mM) of Additives & Reaction Conditions | | | | | |
|---|---|---|---|---|---|
| OMPB (mM) | $FUM-NH_3$ | GLU | pH | Temperature | Amount of L-AMPB produced (mM) |
| 100 | 200 | 20 | 8.5 | 37° C | 80.0 |
| 100 | 200 | 20 | 8.5 | 45° C | 80.7 |
| 100 | 200 | 20 | 8.5 | 60° C | 82.8 |
| 100 | 200 | 20 | 7.5 | 37° C | 59.1 |
| 100 | 200 | 20 | 9.5 | 37° C | 47.7 |
| 100 | 200 | 100 | 8.5 | 37° C | 93.1 |
| 100 | 200 | 200 | 8.5 | 37° C | 96.0 |
| 100 | 200 | 0 | 8.5 | 37° C | 21.8 |
| 100 | 400 | 20 | 8.5 | 37° C | 78.0 |
| 200 | 400 | 20 | 8.5 | 37° C | 96.0 |
| 300 | 600 | 20 | 8.5 | 37° C | 114.9 |
| 100 | 0 | 20 | 8.5 | 37° C | 15.0 |
| 100 | 0 | 100 | 8.5 | 37° C | 45.0 |

In the above table, $FUM-NH_3$ means ammonium fumarate which was made by neutralization of fumaric acid with aqueous ammonia added.

GLU means sodium L-glutamate.

(2) The above procedure (1) of this Example 2 was repeated with increasing the amount of the wet cells E. coli NCTC 8196. However, the cell suspension containing increased amounts of the cells was then added with OMPB, aqueous ammonium fumarate and sodium L-glutamate, followed by incubating the resulting reaction mixture at 37° C and at PH 8.5 under agitation. The test results obtained are tabulated in Table 3 below. From Table 3, it is clear that the increased amount of the microbial cells used and hence the increased amounts of the necessary microbial enzymes used can evidently increase the rate of conversion of OMPB into L-AMPB. It may also be seen that when the process of this invention is carried out under the reaction conditions as indicated in Tables 2 and 3, the production of L-AMPB is achievable commercially advantageously.

Table 3

| Concentrations (mM) of Additives & Reaction Conditions | | | | | |
|---|---|---|---|---|---|
| OMPB | $FUM-NH_3$ | GLU | Amount of Cells (g/10mℓ) | Amount of L-AMPB produced (mM) | Conversion Rate of OMPB (%) |
| 200 | 300 | 20 | 0.467 | 115.0 | 57.5 |
| 200 | 300 | 20 | 0.934 | 157.3 | 78.7 |
| 200 | 300 | 20 | 1.865 | 192.9 | 96.5 |

In the above Table 3, $FUM-NH_3$ and GLU have the same meanings as defined for Table 2.

Example 3

The shake-cultivation of Escherichia coli NCTC 8196 was made in the same manner as in the procedure (1) of Example 2. The resulting culture broth (1.9ℓ ) was centrifuged to collect the intact cells of

the bacterial strain used. The cells were mixed with aqueous OMPB, aqueous ammonium fumarate and aqueous sodium L-glutamate so as to prepare 100 mℓ of a reaction mixture wherein the final concentrations of OMPB, ammonium fumarate and sodium L-glutamate were adjusted to 200 mM, 300 mM and 20 mM, respectively and the pH of the mixture was adjusted to pH 8.5. This reaction mixture was incubated at 37° C 24 hours under gentle agitation while maintaining the pH at 8.5, so that the transamination of OMPB took place. After the completed reaction, the resulting reaction liquid was adjusted to pH 3 by addition of hydrochloric acid and then centrifuged to give about 100 mℓ of the supernatant solution containing L-AMPB produced. The concentration of L-AMPB in this supernatant solution was determined by an amino acid analyzer and was found to be about 190 mM. This supernatant solution was passed through a column of 100 mℓ of Dowex 50W ($H^+$-form; a cation-exchange resin) and then eluted with water. L-AMPB-containing fractions of the eluate were combined and passed through a column of 100 mℓ of Dowex 1 x 2 ($CH_3COO^-$-form; an anion-exchange resin), washed with water and eluted with aqueous 0.3N acetic acid solution. L-AMPB-containing fractions of the eluate were combined and concentrated to dryness. A white powder of L-AMPB obtained was recrystallized from methanol to give ca. 2.4 g of crystals. Analytical data (elementary analysis, specific rotation, melting point, IR spectrum, NMR spectrum and mass spectrum) of the resulting crystalline product of L-AMPB completely coincided with those of an authentic sample of L-AMPB.

As shown hereinabove, the present invention can provide a process capable of producing L-AMPB in large quantities in a more efficient and inexpensive manner, as compared with the prior art method where amino acids are employed as the amino-donor.

## Claims

1. A process for the production of L-2-amino-4-(hydroxymethylphosphinyl)-butyric acid of the formula (I):

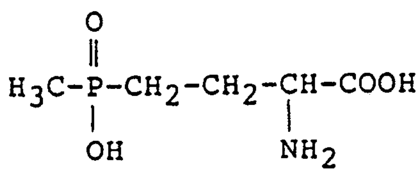

(I)

which comprises transaminating 4-(hydroxymethylphosphinyl)-2-oxo-butyric acid of the formula (II):

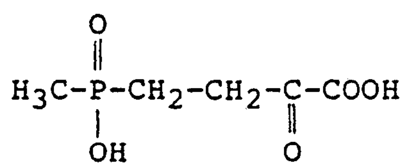

(II)

by reacting with fumaric acid and ammonium ion either in the presence of a culture broth of a microorganism which is capable of producing an aspartase and also capable of producing a transaminase that can effect transamination between 4-(hydroxymethylphosphinyl)-2-oxo-butyric acid and an amino-donor to yield said L-2-amino-4-(hydroxymethylphosphinyl)-butyric acid, or in the presence of separated intact cells of said microorganism, or in the presence of a purified or partially purified enzyme preparation containing said aspartase and transaminase as extracted from the cells of said microorganism.

2. A process of Claim 1 in which 4-(hydroxymethylphosphinyl)-2-oxo-butyric acid is transaminated by reacting with both fumaric acid and ammonium ion as well as with glutamic acid either in the presence of a culture broth of said microorganism or in the presence of intact cells of said microorganism or in the presence of a purified or partially purified enzyme preparation as extracted from the cells of said microorganism.

3. A process of Claim 1 or Claim 2 in which the microorganism is selected from the genus Escherichia, Pseudomonas, Klebsiella, Brevibacterium, Serratia, Proteus or Bacillus.

4. A process of Claim 1 or Claim 2 in which the microorganism is selected from Escherichia coli NCTC

8196 and Escherichia coli NIHJ JC-2 (IFO 12734).

5. A process of Claim 1 or Claim 2 in which the microorganism is selected from Pseudomonas aeruginosa NCTC 10490, Klebsiella pneumoniae ATCC 10031, Seratia marcescens IAM 1223, Proteus vulgaris ATCC 13315 and Bacillus subtilis ATCC 6633.

6. A process of any of claims 1 to 5 in which fumaric acid is present in an equimolar proportion to 4 molar proportion per 1 molar proportion of the starting 4-(hydroxymethylphosphinyl)-2-oxo-butyric acid, and the ammonium ion is present at least in a proportion equimolar to the amount of fumaric acid present.

7. A process of any of claims 2 to 6 in which glutamic acid is present at most in a proportion equimolar to the amount of the starting 4-(hydroxymethylphosphinyl)-2-oxo-butyric acid.

8. A process of any of claims 1 to 7 in which the transamination reaction is carried out at a temperature of 30 °C to 70 °C and under alkaline condition in a range of pH 8 to pH 10 for a time of 15 hours to 48 hours until a substantial amount of L-2-amino-4-(hydroxymethylphosphinyl)-butyric acid is produced and accumulated in the reaction solution.

9. A process for producing L-2-amino-4-(hydroxymethylphosphinyl)-butyric acid, which comprises mixing 4-(hydroxymethylphosphinyl)-2-oxo-butyric acid and water with fumaric acid and ammonium ion and also with intact cells of an aspartase-producing and transaminase-producing microorganism or a purified or partially purified enzyme preparation containing the aspartase and transaminase of said microorganism, optionally together with L-glutamic acid or sodium L-glutamate, and then incubating the resulting mixture at a temperature of 30 °C to 70 °C at pH 8 to pH 10 until 4-(hydroxymethylphosphinyl)-2-oxo-butyric acid is transaminated to produce and accumulate L-2-amino-4-(hydroxymethylphosphinyl)-butyric acid in the incubated mixture, and then recovering L-2-amino-4-(hydroxymethylphosphinyl)-butyric acid from the incubated mixture.

10. A process for producing L-2-amino-4-(hydroxymethylphosphinyl)-butyric acid, which comprises transaminating 4-(hydroxymethylphosphinyl)-2-oxo-butyric acid by reacting it with fumaric acid and ammonium ion together with or without L-glutamic acid or sodium L-glutamate, in the presence of an aspartase and a transaminase which are produced by an aspartase-producing and transaminase-producing microorganism selected from the groups of bacterial strains of the genera Escherichia, Pseudomonas, Klebsiella, Brevibacterium, Serratia, Proteus and Bacillus, to produce L-2-amino-4-(hydroxymethylphosphinyl)-butyric acid.